# EUROPÄISCHE PATENTANMELDUNG

(11) **EP 1 982 920 A1**
(43) Veröffentlichungstag der Anmeldung: **22.10.2008**
(21) Anmeldenummer: 07007977.7
(22) Anmeldetag: 19.04.2007
(51) Int. Cl.: B65B 55/08

(54) **Vorrichtung zum Sterilisieren von Behältnissen**

(71) Anmelder: KRONES AG, 93073 Neutraubling (DE)
(72) Erfinder: Humele, Heinz, 93107 Thalmassing (DE); Dr. Krüger, Jochen, 93107 Thalmassing (DE); Föll, Eberhard, 72147 Nehren (DE); Menz, Hans-Jürgen, 71522 Backnang (DE)
(74) Vertreter: Bittner, Bernhard

(57) **Zusammenfassung**

Eine Vorrichtung (1) zum Sterilisieren von Behältnissen (10) mit einem Behandlungskopf (5) der ein Austrittsfenster (8) aufweist, durch welches Ladungsträger hindurchtreten können, mit einer Ladungsträgererzeugungsquelle, die Ladungsträger erzeugt und mit einer Beschleunigungseinrichtung (6) welche die Ladungsträger in Richtung des Austrittfensters (8) beschleunigt. Erfindungsgemäß ist der Querschnitt des Behandlungkopfes (5) derart beschaffen, dass der Behandlungskopf (5) durch die Mündung des Behältnisses (10) führbar ist und die Beschleunigungseinrichtung (6) beschleunigt die Ladungsträger derart, dass die aus dem Austrittsfenster (8) austretenden Ladungsträger bevorzugt direkt auf eine Innenwandung des Behältnisses (10) richtbar sind.

## Beschreibung

Die vorliegende Erfindung betrifft eine Vorrichtung zum Sterilisieren von Behältnissen, genauer gesagt zum Sterilisieren von Behältnissen vor deren Abfüllung. Speziell soll durch die erfindungsgemäße Vorrichtung die Innenwandung von zu befüllenden Behältnissen, insbesondere von Flaschen, welche eine Mündung aufweisen, sterilisiert werden.

Wenn Lebensmittel in ein Behältnis eingefüllt werden, ist es erforderlich, das Behältnis selbst zu sterilisieren. So ist es beispielsweise bekannt, die Innenwandung von Behältnissen mit Dampf oder Wasserstoffperoxid zu sterilisieren. Derartige Verfahren sind jedoch mit Nachteilen verbunden, da es durch die Behandlung beispielsweise mit Wasserstoffperoxid zu einer Aufweichung des Materials kommen kann. Daher ist es aus dem Stand der Technik seit langen bekannt, die Behältnisse mit Hilfe von Ladungsträgern wie insbesondere Elektronenstrahlen zu sterilisieren.

Aus der JP 2002-255125 ist eine Vorrichtung zum Sterilisieren von Behältnissen bekannt. Dabei ist eine außerhalb des Behältnisses vorgesehene Strahlungsquelle vorgesehen, die Strahlung in das Innere des Behältnisses richtet. Auch in der JP 2001-225814 wird eine entsprechende Vorrichtung zur Innenwandsterilisation von Behältnissen beschrieben, bei der eine Strahlungsquelle von außerhalb Strahlung in das Behältnis einstrahlt.

Aus der FR 2 815 769 ist eine Elektronenstrahlquelle bekannt, die zwei Elektronenbündel ausstrahlt.

Die DE 198 82 252 T1 beschreibt eine Technik zur Innensterilisation eines Behälters mittels Elektronen. Dabei ist ebenfalls eine Elektronenstrahlquelle vorgesehen, die Strahlung von außerhalb in das Innere des Behältnisses richtet.

Diese genannten Vorrichtungen weisen den Nachteil auf, dass die Strahlung stets von außen durch die Mündung des Behältnisses in dieses eindringt und damit im Inneren des Behältnisses nur schwer hinsichtlich ihrer Strahlrichtung variiert werden kann.

Aus der EP 0 885 142 B1 ist ein Verfahren zur Sterilisation von Fliesmittelpackungen bekannt. Dabei handelt es sich insbesondere um Verpackungen mit einer offenen Seite. Bei diesem Verfahren sollen hohe Beschleunigungsspannungen vermieden werden, da als unerwünschter Nebeneffekt dieser hohen Beschleunigungsspannungen Röntgenstrahlen erzeugt werden und diese wiederum mit einem Bleimantel abgeschirmt werden müssten. Um gleichermaßen einen Innenreinigungseffekt zu erreichen, wird in der EP 0 885 142 B1 vorgeschlagen, einen Gasstrom zu verwenden, der mit einem Elektronenstrahl in Kontakt steht und auf diese Weise dazu beiträgt, dass die Elektronen an die Innenwandung des Behältnisses gelangen können. Das in der EP 0 885 142 B1 beschriebene Verfahren ist jedoch zur Innenreinigung von Flaschen oder allgemein von Behältnissen mit einem Mündungsdurchmesser ungeeignet, da die in der EP 0 885 142 B1 beschriebene Elektronenstrahlquelle nicht durch diese Mündung hindurchgeführt werden könnte und jedenfalls nicht gemeinsam mit dem in der EP 0 885 142 B1 beschriebenen Luftrohr, welches den Luftstrom im Inneren des Behältnisses erzeugt, in dieses Behältnis eingeführt werden könnte.

Der vorliegenden Erfindung liegt daher die Aufgabe zugrunde, eine effiziente Vorrichtung und ein Verfahren zur Innenreinigung von Behältnissen mit im Vergleich zu dem Behältnisquerschnitt engerem Mündungsquerschnitt zur Verfügung zu stellen.

Dies wird erfindungsgemäß durch eine Vorrichtung nach Anspruch 1, eine Anordnung nach Anspruch 15 und ein Verfahren nach Anspruch 20 erreicht. Vorteilhafte Ausführungsformen und Weiterbildungen sind Gegenstand der Unteransprüche.

Die erfindungsgemäße Vorrichtung zum Sterilisieren von Behältnissen weist einen Behandlungskopf auf, der wiederum ein Austrittsfenster aufweist, durch welches Ladungsträger hindurchtreten können. Weiterhin ist eine Ladungsträgererzeugungsquelle vorgesehen, die Ladungsträger erzeugt und eine Beschleunigungseinrichtung, welche die Ladungsträger in Richtung des Austrittsfensters beschleunigt.

Erfindungsgemäß ist der Querschnitt des Behandlungskopfes derart beschaffen, dass der Behandlungskopf durch die Mündung des Behältnis führbar ist und die Beschleunigungseinrichtung beschleunigt die Ladungsträger derart, dass die aus dem Austrittsfenster austretenden Ladungsträger bevorzugt direkt auf eine Innenwandung des Behältnisses richtbar sind.

Bei den Ladungsträgern handelt es sich insbesondere um Elektronen, es wäre jedoch auch denkbar, dass andere Ladungsträger, wie Ionen, verwendet werden.

Im Gegensatz zu dem zitierten Stand der Technik werden damit Beschleunigungsspannungen verwendet, die hoch genug sind, dass der austretende Elektronenstrahl direkt auf die Innenwandung des Behältnisses auftrifft, ohne dass durch Zwischenschaltung eines Gasstroms dieser durch den Elektronenstrahl für eine sterilisierende Wirkung aktiviert wird. Unter einen Austrittsfenster wird eine Einrichtung verstanden, welche zwar den Innenraum der Vorrichtung, d. h. den Bereich zwischen der Ladungsträgererzeugungsquelle und dem Austrittsfenster luftdicht abschließt, durch die jedoch gleichwohl die Ladungsträger insbesondere die Elektronen durchtreten können. Es ist zu beachten, dass die aus dem Austrittsfenster austretenden Elektronen stark gebremst werden, sodass im Gegensatz zu der aus der EP 0 885 142 B1 bekannten Vorrichtung signifikant höhere Energien bzw. Beschleunigungsspannungen verwendet werden.

Zur Erzeugung des Elektronenstrahls dient bevorzugt eine kompakte Elektronenstrahleinheit mit einem Strahlfinger der, wie oben erwähnt, so dimensioniert ist, dass er in die Flasche eintauchen kann um damit eine Elektronenwolke mit möglichst geringer Energie auf die innere Oberfläche der (PET)-Flaschen aufzubringen. Im Falle einer Rundläuferanordnung wäre es möglich, dass Betriebseinheiten für die Vorrichtung, wie beispielsweise Transformatoren oder Netzgeräte für die Elektronenstrahlerzeuger auf einen Karussell mitlaufen, wodurch gleichzeitig die Zuführung von Hochspannung vereinfacht wird. Bei einer bevorzugten Ausführungsform sind das Austrittsfenster und die Beschleunigungseinrichtung innerhalb eines Außengehäuses angeordnet und dieses Außengehäuse ist derart beschaffen, dass es durch die Mündung des Behältnisses führbar ist. Der Behandlungskopf ist damit am unteren Ende dieses Außengehäuses vorgesehen bzw. es ist auch möglich, dass der Behandlungskopf mit dem Außengehäuse einteilig ausgebildet ist. Bevorzugt weist damit das gesamte Außengehäuse mit dem Behandlungskopf einen Querschnitt auf, der durch die Mündung eines Behältnisses einführbar ist. Der Behandlungskopfquerschnitt kann beliebig ausgeformt sein. Besonders bevorzugt weist die Vorrichtung einen kreisförmigen Querschnitt mit einem Durchmesser auf, der unter 40 mm, bevorzugt unter 30 mm und besonders bevorzugt unter 25 mm liegt.

Bevorzugt ist die gesamte Vorrichtung von einer Abschirmungseinrichtung umgeben und insbesondere von einem Bleimantel, um den Austritt von Röntgenstrahlung und damit schädliche Wirkungen auf die Benutzer zu verhindern.

Bei einer weiteren bevorzugten Ausführungsform weist die Vorrichtung ein Innengehäuse auf, innerhalb dessen die Beschleunigungseinrichtung angeordnet ist und das Außengehäuse umgibt dieses Innengehäuse. Der Innenraum dieses Innengehäuses, in dem bevorzugt die Ladungsträgererzeugungsquelle und die Beschleunigungseinrichtung vorgesehen sind, werden besonders bevorzugt mit einem Vakuum beaufschlagt. Besonders bevorzugt wird zwischen dem Außengehäuse und dem Innengehäuse ein sich bis zu dem Behandlungskopf erstreckender Raum gebildet, durch den ein Medium und insbesondere ein gasförmiges Medium führbar ist. Dieses gasförmige Medium dient insbesondere zum Kühlen des Austrittfensters und zu diesem Zweck wird das gasförmige Medium entlang des Innengehäuses bis zu dem Behandlungskopf und auch vorbei an dem Austrittsfenster geführt. Vorzugsweise wird damit zwischen dem Außengehäuse und dem Innengehäuse ein Raum in Form eines Kanals oder mehrerer Kanäle gebildet, wobei besonders bevorzugt ein unterer Endabschnitt des äußeren Gehäuses so gestaltet ist, dass der Gasstrom auch beispielsweise in radialer Richtung der Vorrichtung und damit auch an dem Austrittsfenster vorbeigeführt wird. Es wird darauf hingewiesen, dass der Strom des gasförmigen Mediums nicht und zumindest nicht unmittelbar an die Innenwandung des Behältnisses gelangt, sondern, wie gesagt, insbesondere dazu dient, um an dem Austrittsfenster vorbeigeführt zu werden.

Vorteilhaft ist das gasförmige Medium aus einer Gruppe von gasförmigen Medien ausgewählt, welche Helium, Stickstoff, Argon, Kohlendioxid, Mischungen hieraus oder dergleichen enthält.

Das Austrittsfenster ist besonders bevorzugt aus einem Material hergestellt, welches aus einer Gruppe von Materialien ausgewählt ist, welche Titan, Quarzglas, Diamant, Kombinationen hieraus und dergleichen enthält.

Damit ist bei dieser Ausführungsform zumindest ein Teil der Vorrichtung doppelwandig ausgeführt, wobei zwischen dem Außengehäuse und dem Innengehäuse ein Gas oder Luft zur Kühlung des Stahlrohres, d. h. des Innengehäuses und des Fensters ermöglicht wird. Wie erwähnt ist das Innere des Innengehäuses evakuiert und trägt bevorzugt als Austrittsfenster am Ende ein Titanfenster. Bei einer weiteren bevorzugten Ausführungsform weist das Austrittsfenster eine Dicke auf, welche zwischen 3 µm und 30 µm, bevorzugt zwischen 4 µm und 25 µm und besonders bevorzugt zwischen 5 µm und 20 µm liegt. Damit ist es möglich, für das Austrittfenster eine Fensterfolie beispielsweise aus Titan mit Dicken von 8 µm, 10 µm, 13 µm bzw. 15 µm zu verwenden. Es kann jedoch auch ein anderes geeignetes Material verwendet werden. Dieses Austrittsfenster bzw. die Fensterfolie wird mit dem inneren Gehäuse, welches ebenfalls aus geeignetem Material (wie z. B. ebenfalls aus Titan) besteht, vakuumdicht verschweißt. Dabei ist es möglich, dass das Austrittsfenster freitragend über die Öffnung des Innengehäuses gespannt ist, es ist jedoch auch möglich, dass eine Stützkonstruktion wie beispielsweise eine Lochplatte vorgesehen ist, welche das Austrittsfenster trägt. Im Fall der Verwendung einer Stützkonstruktion für das Austrittsfenster ist es auch denkbar, dass diese Stützkonstruktion durch eine geeignete Flüssigkeit gekühlt wird, die durch einen oder mehrere Kanäle zwischen dem Innengehäuse und dem Außengehäuse zur Stützkonstruktion hin und wieder abgeführt wird.

Bei einer bevorzugten Ausführungsform weist das Austrittsfenster über seine Fläche hinweg eine gleichmäßige Dicke auf. Es wäre jedoch auch je nach Anwendungsfall denkbar, dass die Dicke variiert, beispielsweise von außen nach innen zunimmt. So ist es möglich, dass die Fensterfolie auch über ihre Fläche hinweg unterschiedliche Dicken aufweist beispielsweise schwankend zwischen 4 und 13 µm oder auch in Form einer Dickenperforation in Linien oder Punkten. Auf diese Weise ist es möglich, unterschiedlich schnelle Elektronen an der Atmosphäre, d. h. der Außenseite des Austrittsfensters zu erhalten und damit ein von einem üblichen Streufeld abweichendes Streufeld.

Wie oben erwähnt, können als Kühlgas unterschiedliche Gase eingesetzt werden. Auf diese Weise kann eine verbesserte Wärmeleitung der Gase erreicht werden, insbesondere mit Helium aber es wäre auch möglich, die Bedingungen für die Elektronen an der Atmosphäre d. h. nach dem Austritt aus dem Austrittsfenster zu beeinflussen. So führt beispielsweise die Verwendung von Helium als Atmosphäre zu größeren Reichweiten der Elektronen, da Helium eine geringere Dichte aufweist als Luft. Weiterhin kann durch die Zuführung der Gase auch eine Inertisierung des Behandlungsraums d. h. des Innenraums des Behältnisses, erreicht werden, was wiederum zu einer Verminderung der Ozonentstehung führt.

Als Strahlerzeuger kann sowohl eine offene als auch eine geschlossene Elektronenstrahleinheit, die beispielsweise aus Röntgenröhren oder ähnlichem bekannt ist, verwendet werden. Die Elektronenquelle selbst kann aus punktförmige oder flächige Elektronenquelle ausgeführt sein, an die das Innengehäuse bzw. der Strahlfinger direkt angebracht ist.

Die erfindungsgemäße Vorrichtung wird im Folgenden auch als Strahlfinger bezeichnet.

Bei einer weiteren vorteilhaften Ausführungsform weist die Vorrichtung eine Ablenkeinheit zum Ablenken der Ladungsträger auf. Besonders bevorzugt ist diese Ablenkeinrichtung zwischen der Ladungsträgererzeugungsquelle und dem Austrittsfenster angeordnet. Damit kann eine elektromagnetische Ablenkung der Elektronen vor dem Austrittfenster stattfinden, um den Elektronen zusätzlich eine Vorzugsrichtung in Richtung der Flaschenwand zu geben. Auf diese Weise ist es möglich, die Strahlungsdosis gleichmäßiger über die Innenoberfläche des Behältnisses zu verteilen. Auch ist es auf diese Weise möglich, den Elektronenstrahl gleichmäßig über die gesamte Austrittsfläche des Austrittsfensters zu führen um auf diese Weise eine gleichmäßigere Belastung des Austrittfensters in seiner Gesamtheit zu erreichen bzw. um damit sog. Hotspots auf dem Fenster zu verhindern. Es ist jedoch auch denkbar, dass die Ablenkeinheit in Richtung des Elektronenstrahls nach dem Austrittsfenster angeordnet ist.

Bevorzugt weist das Innengehäuse im Bereich des Behandlungkopfes einen vergrößerten Innendurchmesser auf, um diese erwähnte Ablenkung der Ladungsträger zu vereinfachen.

Weiterhin ist besonders bevorzugt der Behandlungskopf gegenüber den Behältnissen in einer Längsrichtung des Behältnisses bewegbar. Auf diese Weise kann in einem Arbeitsgang ein größerer flächiger Bereich der Innenwandung des Behältnisses sterilisiert werden.

Bei einer weiteren vorteilhaften Ausführungsform ist der Behandlungskopf gegenüber dem Behältnis drehbar. Dies ist insbesondere dann vorteilhaft, wenn die Ladungsträger auch in einer bevorzugten radialen Richtung bezüglich des Behandlungskopfes austreten. Bei einer weiteren vorteilhaften Ausführungsform ist der Behandlungskopf bezüglich dem Behältnis in einer radialen Richtung des Behältnisses bewegbar. So ist es beispielsweise möglich, dass während des Sterilisationsvorgangs der Behandlungskopf selbst bzw. die Vorrichtung um eine vorgegebene Schwenkachse geschwenkt wird oder auch, dass das Behältnis selbst gegenüber dem Behandlungskopf geschwenkt wird. Besonders bevorzugt ist dabei eine Schwenkachse in einem Bereich der Mündung des Behältnisses bzw. geringfügig oberhalb der Mündung des Behältnisses vorgesehen. Auf diese Weise kann der Behandlungskopf in die Nähe der Innenwandung des Behältnisses gebracht werden.

Die vorliegende Vorrichtung ist weiterhin auf eine Anordnung zum Behandeln von Behältnissen mit wenigstens einer Vorrichtung der oben beschriebenen Art gerichtet. Bevorzugt sind mehrere der oben beschriebenen Vorrichtungen nebeneinander bzw. hintereinander angeordnet, um mehrere Behältnisse gleichzeitig sterilisieren zu können. Es ist beispielsweise möglich, die gesamte Anordnung als Rundläufer oder Lineareinheit auszuführen, wobei ein Behandlungsraum vorgesehen ist, der der Innensterilisation von Getränkeflaschen durch Elektronenstrahlen dient. So ist es auch möglich, die erfindungsgemäße Vorrichtung in eine Streckblasmaschine oder eine Fülleinrichtung zu integrieren oder auch als allein stehende Einheit anzuordnen, um bereits bestehende Linien oder Anordnungen nachrüsten zu können.

Vorzugsweise weist die Anordnung eine Einrichtung zum Befüllen von Behältnissen auf und die erfindungsgemäße Vorrichtung ist stromaufwärts bezüglich dieser Einrichtung angeordnet.

Weiterhin kann die Anordnung eine Verschiebeeinrichtung aufweisen, welche die Behältnisse in der Längsrichtung der Behältnisse gegenüber der Vorrichtung verschiebt. Grundsätzlich wäre es auch möglich, die Höhenlage der Behältnisse beizubehalten und lediglich die Vorrichtung in der Längsrichtung der Behältnisse zu verschieben. Da jedoch die Vorrichtung bzw. der Strahlfinger relativ aufwendig gestaltet und üblicherweise auch mit Hochspannungen beaufschlagt sind, bietet es sich eher an, diese stationär zu halten und vielmehr die Behältnisse gegenüber der Vorrichtung zu verschieben.

Weiterhin ist vorteilhaft die Vorrichtung zwischen einer Expansionseinrichtung für die Behältnisse und einer Einrichtung zum Befüllen der Behältnisse angeordnet. Damit wird eine Sterilisation dieser Anordnung nach dem Expandieren der Behältnisse bzw. den Blasen der Behältnisse durchgeführt.

Vorteilhaft weist die Anordnung eine Vielzahl von Vorrichtungen der oben beschriebenen Art auf, wobei diese einzelnen Vorrichtungen besonders bevorzugt entlang einer Kreisbahn angeordnet sind. Es wäre jedoch auch möglich, eine Vielzahl von Vorrichtungen entlang einer im Wesentlichen geraden Strecke beispielsweise parallel zu einem in gerader Richtung verlaufenden Transportband anzuordnen.

Die vorliegende Erfindung ist weiterhin auf ein Verfahren zum Sterilisieren von Behältnissen gerichtet, wobei in einer Vorrichtung zum Sterilisieren der Behältnisse Ladungsträger erzeugt und in Richtung eines Austrittfensters beschleunigt werden, wobei dieses Austrittsfenster in einem Behandlungskopf angeordnet ist. Erfindungsgemäß wird der Behandlungskopf der Vorrichtung durch eine Mündung des Behältnisses in das Innere des Behältnisses eingeführt und ausgehend von den Behandlungskopf werden beschleunigte Ladungsträger direkt auf die Innenwandung des Behältnisses gerichtet und das Behältnis bevorzugt relativ zu dem Behandlungskopf bewegt.

Vorzugsweise werden die Ladungsträger vor Erreichen des Austrittfensters in einer radialen Richtung des Behältnisses bzw. der Vorrichtung abgelenkt. Zu dieser Ablenkung können Spulen oder dergleichen eingesetzt werden.

Weitere Vorteile und Ausführungsformen ergeben sich aus den beigefügten Zeichnungen:
Darin zeigen:
   - Fig. 1a: eine teilweise Ansicht einer erfindungsgemäßen Vorrichtung zum sterilisieren von Behältnissen;
   - Fig. 1b: ein Außengehäuse für die Vorrichtung aus Fig. 1a;
   - Fig. 1c: ein Innengehäuse für die Vorrichtung aus Fig. 1a;
   - Fig. 1d: einen Stützkörper mit Austrittsfenster für die Vorrichtung aus Fig. 1a;
   - Fig. 1e: eine Draufsicht auf den Stützfenster aus Fig. 1d;
   - Fig. 1f: eine Draufsicht auf das Austrittsfenster aus Fig. 1a;
   - Fig. 1g: eine weitere Ausführungsform eines Stützfensters;
   - Fig. 2: eine weitere Ausführungsform einer erfindungsgemäßen Vorrichtung;
   - Fig. 3: eine weitere Ausführungsform einer erfindungsgemäßen Vorrichtung;
   - Fig. 4: eine Anordnung zum Behandeln von Behältnissen;
   - Fig. 5: ein weiteres Beispiel für eine Anordnung zum Behandeln von Behältnissen;
   - Fig. 6: eine Detailansicht einer Anordnung zum Behandeln von Behältnissen;
   - Fig. 7: ausschnittsweise schematische Seitenansicht einer Anordnung zum Behandeln von Behältnissen und
   - Fig. 8: eine weitere Ausführungsform einer erfindungsgemäßen Vorrichtung.

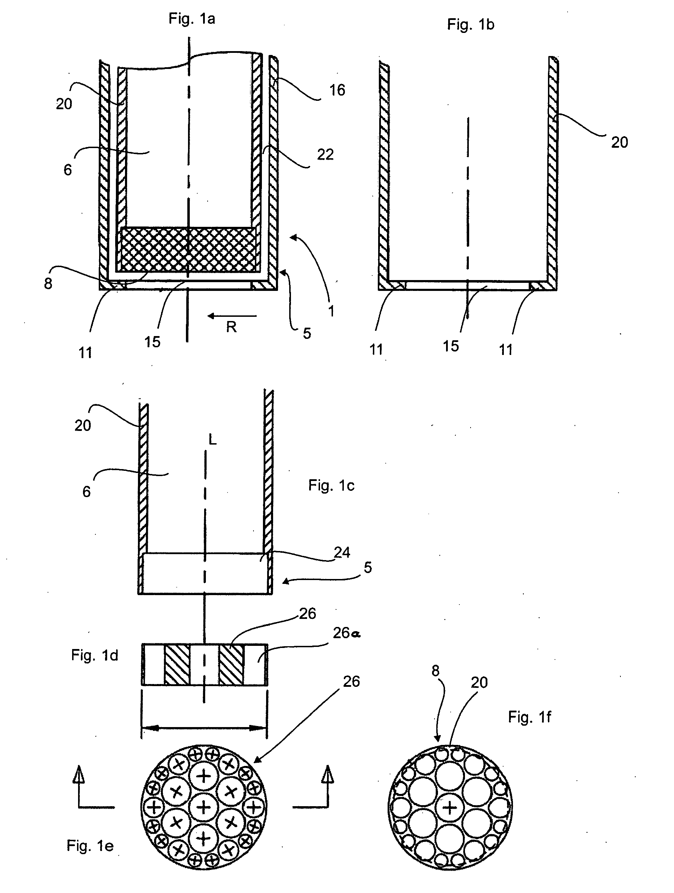

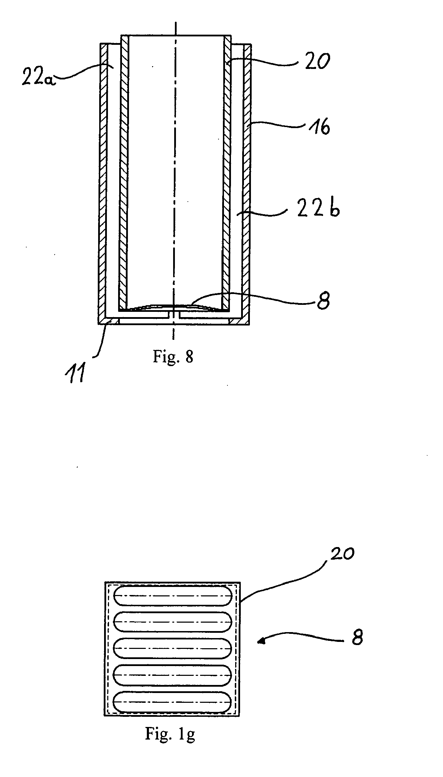

Fig. 1a zeigt eine ausschnittsweise Darstellung einer erfindungsgemäßen Vorrichtung zum Sterilisieren von Behältnissen. Diese Vorrichtung weist an ihrem unteren Ende einen Behandlungskopf 5 auf, an dem ein Austrittsfenster 8 vorgesehen ist, über welches ein Elektronenstrahl austreten kann. Dabei werden, wie aus dem Stand der Technik üblich, zunächst beispielsweise mit Hilfe einer Wolframkathode die Elektronen erzeugt. Diese Elektronen werden anschließend über eine (nicht im Detail gezeigte) Beschleunigungseinrichtung 6 beschleunigt. Als Elektronenerzeugungsquelle können dabei punktförmige oder auch flächige Elektronenquellen verwendet werden.

Die Vorrichtung 1 zum Sterilisieren von Behältnissen weist ein Außengehäuse 16 und ein Innengehäuse 20 auf, ist also doppelwandig ausgeführt. Zwischen dem Außengehäuse 16 und dem Innengehäuse 20 ist ein Spalt 22 gebildet, entlang dessen Luft, ein anderes gasförmiges Medium oder auch ein Flüssiges Medium geführt werden kann. Dabei kann dieser Luftspalt 22 in Umfangsrichtung umlaufend ausgebildet sein, es ist jedoch auch möglich, dass eine Vielzahl von Kanälen 22 vorgesehen ist. Prinzipiell ist es dabei möglich, dass der Gasstrahl während des Betriebs der Vorrichtung 1 in radialer Richtung R an dem Austrittsfenster 8 vorbeigeführt wird, es ist jedoch auch möglich, dass der Gasstrom in dem Zeitraum an dem Austrittsfenster 8 vorbeigeführt wird, in dem die Vorrichtung 1 nicht aktiv ist bzw. die Strahlungsquelle nicht aktiv ist. Auf diese Weise kann verhindert werden, dass der austretende Elektronenstrahl durch den Luftstrom beeinflusst wird. Es wird dabei darauf hingewiesen, dass der Gasstrom ausschließlich zum Kühlen des Austrittfensters dient und nicht zum Führen des Elektronenstrahls.

Grundsätzlich ist der Strahlstrom im Falle einer optimal gewählten Beschleunigungsspannung der maßgebenende Faktor, um in möglichst kurzer Zeit die entsprechend notwendige Dosis im Behältnis zu erzeugen. Dieser Strahlstrom führt jedoch in dem Austrittsfenster 8 zu Verlusten, die je nach Ausführung dieses Austrittsfensters 8 früher oder später auch die maximale Strahlleistung der Elektronenstrahleinheit bzw. der Vorrichtung 1 begrenzen. Mit der beschriebenen Luft-, Gas- oder Flüssigkeitskühlung kann jedoch auch die notwendige Kühlung des Austrittsfensters gewährleistet werden. Mit anderen Worten soll, um den maximal möglichen Strahlstrom zu erreichen d. h. den maximal möglichen Durchsatz, die Anzahl der Strahleinheiten minimiert bzw. die Taktzeit erhöht werden. Auch wäre es möglich, die Streugeometrie an der Atmosphäre d. h. außerhalb des Austrittsfensters 8 zu verbessern.

Die Elektronen werden auf Energie in einen Bereich von 100 keV - 200 keV vorzugsweise zwischen 120 keV und 180 keV und vorzugsweise 130 keV und 170 keV beschleunigt.

Fig. 1b zeigt ein Außengehäuse 20 für eine erfindungsgemäße Vorrichtung 1. Man erkennt, dass am unteren Ende des Außengehäuses 20 in radialer Richtung nach innen ragende Wände 11 vorgesehen sind. Diese Wände 11 dienen zur Luft bzw. Gasführung d. h. sie bewirken, dass Gas wenigstens auch abschnittsweise Austrittsfenster 8 vorbeigeführt werden. Durch diese Wände 11 wird weiterhin eine Öffnung 15 gebildet, durch welche auch der entstehende Elektronenstrahl nach außen treten kann.

Es wäre jedoch auch möglich, auf einer Seite bezüglich des Innengehäuses 20 Gas zuzuführen und dieses auf der anderen Seite des Innengehäuses 20 wieder abzuführen.

Fig. 1c zeigt ein Innengehäuse 20. Dieses Innengehäuse 20 weist an seinem unteren Ende bzw. an dem Behandlungskopf 5 eine Ausnehmung 24 auf, um das Austrittsfenster 8 bzw. auch einen Stützkörper 26 des Austrittsfensters 8 aufzunehmen.

Fig. 1d zeigt einen Stützkörper 26, der zum Stützen des eigentlichen in Fig. 1f gezeigten Austrittsfensters 8 dient. Bei dieser Ausführungsform bildet dieser Stützkörper 26 eine Vielzahl von Kanälen 26a, durch welche hindurch der Elektronenstrahl treten kann. Zusätzlich kann der Stützkörper 26 Kanäle aufweisen, durch die hindurch eine Kühlfüssigkeit oder ein Kühlgas geführt werden kann.

Das in Fig. 1f gezeigte eigentliche Austrittsfenster 8 ist beispielsweise auf den Stützkörper 26 angeschweißt.

Für das Innengehäuse 20 bzw. die gesamte Vorrichtung 1 sind unterschiedliche Ausführungsformen denkbar. So ist es möglich, die Vorrichtung beispielsweise aus Quarzglas auszuführen, d. h. insbesondere das Innenrohr 20 des Quarzglases auszuführen. Dabei kann beispielsweise eine dünne Quarzglasfolie als Austrittsfenster 8 eingeschmolzen sein. Aus dem Stand der Technik sind bereits derartige Folien mit einer Dicke von 20 µm bekannt. Die Dichte derartiger Fenster liegt in einem Bereich von 2,2 g/cm³ bzw. entsprechend 44 g/cm². Dies entspricht der Flächendichte einer Titanfolie mit einer Dicke von 10 µm.

Derartige Quarzglasfolien erlauben Betriebtemperaturen von bis 1000° C d. h. zur Kühlung ist auch ein hoher Temperaturgradient möglich. Ein weiterer Vorteil derartiger Glasfolien aus Quarzglas ist, das Quarzglasfolien in dieser Dicke elastisch sind.

Eine weitere Möglichkeit bestünde darin, die Vorrichtung 1 bzw. das Innengehäuse 20 aus Quarzglas auszuführen und das Austrittsfenster 8 aus Diamant. Auch hier sind bereits Folien mit einer Dicke von 10 µm bekannt. Derartige Folien weisen eine Dichte im Bereich von 3,5 g/cm³ bzw. 35 g/cm² auf. Der Vorteil derartiger Folien aus Diamant sind eine weiter verbesserte Wärmeleitfähigkeit, die beispielsweise die Wärmeleitfähigkeit von Kupfer um den Faktor 5 übertrifft.

Weiterhin wäre es auch möglich, die Vorrichtung 1 d. h. insbesondere das Innenrohr bzw. Innengehäuse 20 aus Metall (z. B. Titan) auszuführen und das Austrittsfenster 8, wie oben erwähnt, aus Diamant mit den oben bezeichneten Eigenschaften. Dabei wäre es möglich, dass das Austrittsfenster 8 eingelötet oder verschmolzen ist. Falls Glasrohre verwendet werden, sollten diese vorteilhaft metallisiert werden, da über die vergleichsweise lange Strecke Aufladungen auftreten können.

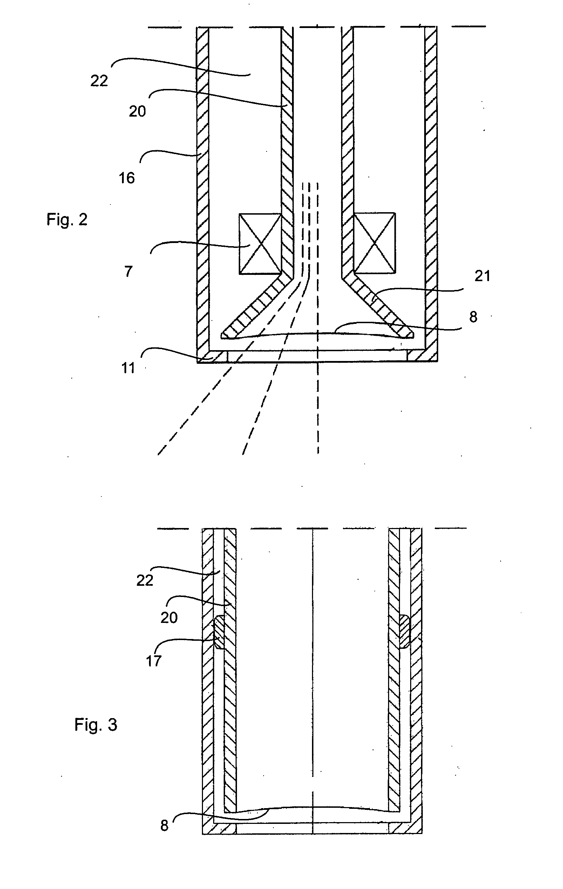

Fig. 2 zeigt eine weitere Ausführungsform einer erfindungsgemäßen Vorrichtung 1. In diesem Falle weist das Innengehäuse 20 bzw. Innenrohr im oberen Bereich einen relativ kleinen Innenquerschnitt auf, der sich im Bereich des Behandlungskopfes 5 erweitert. Auf diese Weise wird ein relativ großer Raum 22 zwischen dem Innengehäuse 20 und dem Außengehäuse 16 gebildet. Im Bereich des Behandlungskopfes 5 weist das Innengehäuse einen sich kegelförmig erweiterten Endabschnitt 21 auf. Durch diesen Endabschnitt 21 ist es möglich, die Elektronenstrahlen unter relativ hohem Winkel zu lenken. Zur Ablenkung des Elektronenstrahls können beispielsweise Ablenkspulen 7 vorgesehen sein. Da neben dem in Fig. 2 gezeigten Spulenpaar noch ein weiteres senkrecht stehendes Spulenpaar angeordnet ist, können die Elektronenstrahlen im Prinzip in alle Raumrichtungen ausgesandt werden. Bei der in Fig. 2 gezeigten Ausführungsform ist lediglich ein Austrittsfenster 8 in Form einer Titanfolie vorgesehen. Es könnte jedoch auch hier ein Stützkörper 26 wie in den Figuren 1a bzw. 1e vorgesehen sein.

Anstelle der in Fig. 2 gezeigten kegelförmigen Erweiterung könnte jedoch der untere Bereich 21 auch beispielsweise halbkugelförmig ausgebildet sein. Weiterhin wäre es jedoch auch möglich, dass die Ablenkspulen außerhalb des äußeren Gehäuses 16 angeordnet sind. Daneben wäre es auch möglich, die Ablenkspulen erst unterhalb der Vorrichtung 1 anzuordnen. Bei der in Fig. 2 gezeigten Ausführungsform ist auch das Innengehäuse 20 aus Titan gebildet.

Fig. 3 zeigt eine weitere Ausführungsform für eine erfindungsgemäße Vorrichtung. Bei dieser Vorrichtung ist ein relativ dünner Luftspalt 22 zwischen dem Innengehäuse 20 und dem Außengehäuse 16 vorgesehen. Um Kontakte zwischen dem Innengehäuse 20 und dem Außengehäuse 16 zu vermeiden, ist es möglich, zwischen den beiden Gehäusen 16, 20 Abstandskörper 17 anzuordnen, wobei die Abstandskörper 17 vorteilhaft elektrisch isoliert sind. Wie bei der in Fig. 2 gezeigten Ausführungsform könnten auch bei der in Fig. 3 gezeigten Ausführungsform Ablenkspulen 7 zur Ablenkung des Elektronenstrahls vorgesehen sein.

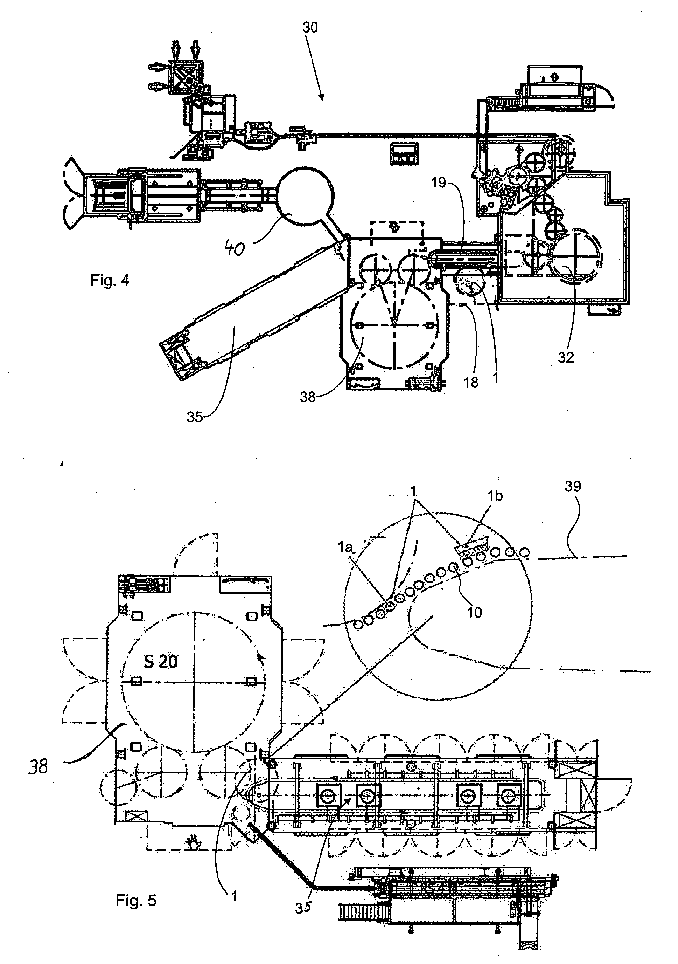

Fig. 4 zeigt eine erfindungsgemäße Anordnung zum Behandeln von Behältnissen. Diese Anordnung weist Sortiereinrichtung 40 für Vorformlinge, vorzugsweise eine als rotierende Scheibe ausgebildete Sortiereinrichtung 40 sowie eine Heizstrecke bzw. einen Ofen 35 auf, in dem Vorformlinge erwärmt werden. An diese Aufwärmstrecke schließt sich eine Blasvorrichtung 38 an, welche die Vorformlinge zu Behältnissen bläst. Im Anschluss auf diese Blasvorrichtung befindet sich eine bzw. eine Vielzahl von erfindungsgemäßen Vorrichtungen 1 zum Sterilisieren der bereits geblasenen Behältnisse. Dabei bezieht sich das Bezugszeichen 18 auf eine Abschirmeinrichtung, wie einen Bleimantel, der die gesamte Vorrichtung 1 umgibt, um den Austritt von Röntgenstrahlen zu verhindern.

Das Bezugszeichen 19 bezieht sich auf ein Transportelement, welches die Behältnisse von der Blasvorrichtung 38 zu einer Fülleinrichtung 32 transportiert. Damit sind in der Bewegungsrichtung der Behältnisse zunächst eine Blasvorrichtung 38 anschließend eine Fülleinrichtung 32 und möglicherweise anschließend noch eine Etikettiermaschine vorgesehen. Es wäre jedoch auch möglich, dass auf die Blasvorrichtung 38 zunächst eine Etikettiermaschine und anschließend eine Fülleinrichtung 32 folgt. Bei der erfindungsgemäßen Anordnung ist die Vorrichtung 1 jeweils unmittelbar nach der Blasvorrichtung 38 angeordnet.

Es wäre weiterhin möglich, neben der erfindungsgemäßen Vorrichtung 1 noch andere ähnlich geartete Sterilisationsvorrichtungen vorzusehen, welche zu der Außenreinigung d. h. der Sterilisation der Außenwände der Behältnisse dienen. Die einzelnen Behältnisse können zur Innensterilisation auf einem Rundläufer geführt werden und dann jeweils die erfindungsgemäßen Vorrichtungen in die Behältnisse eingetaucht werden. Dabei ist es, wie oben erwähnt, vorteilhaft, die einzelnen Vorrichtungen bzw. Strahlfinger auf einem konstanten Höhenniveau zu halten und die Behältnisse diesen gegenüber zu bewegen.

Mit anderen Worten werden die Strahlfinger 1 für die Innensterilisation in die Behältnisse eingetaucht. besonders bevorzugt werden die einzelnen Behältnisse rotierend an einzelnen erfindungsgemäßen Vorrichtungen vorbeigefahren. Es ist jedoch zusätzlich auch möglich, die einzelnen Behältnisse an einem Vorhang mit Elektronenstrahlen vorbeizufahren, um auf diese Weise eine Außensterilisation zu bewirken.

Fig. 5 zeigt eine Detailansicht für eine weitere erfindungsgemäße Anordnung einer Sterilisationsvorrichtung.

Grundsätzlich ist es möglich, dass die Behältnisse über eine Kette transportiert bzw. über eine Kette in einen Behandlungsraum geführt werden und dort Strahlfinger über eine Hubbewegung in die Flaschen eintauchen. Es wäre jedoch auch möglich, die Behältnisse über einen Stern im Neckhandling an eine Sterilisationseinrichtung zu übergeben und dort über eine Hubbewegung über den Strahlfinger zu bewegen.

Bei der in Fig. 5 gezeigten Ausführungsform erfolgt zunächst durch eine Bestrahlungsvorrichtung 1b eine Außenbestrahlung der Behältnisse, bevorzugt während einer Rotation derselben. Dabei werden zur Bestrahlung jeweils auf ca. 150 keV beschleunigte Elektronenstrahlen verwendet. Anschließend wird durch die Vorrichtung 1a in der oben beschriebenen Weise eine Innenbestrahlung der Behältnisse durchgeführt.

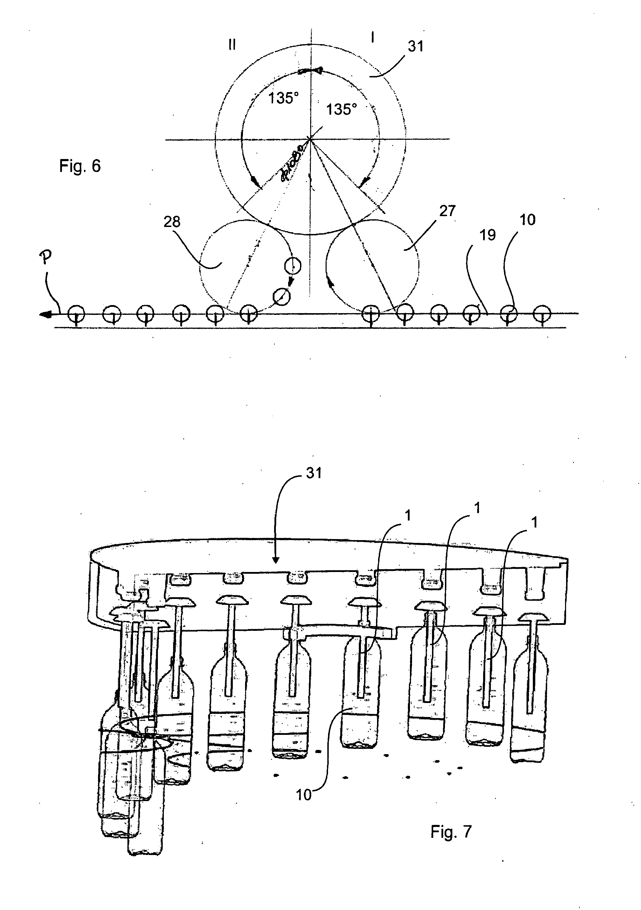

Fig. 6 zeigt eine schematische Veranschaulichung eines erfindungsgemäßen Verfahrens für die Innensterilisation von Behältnissen 10. Die Behältnisse 10 werden über ein Transportband 19 in Richtung des Pfeils P antransportiert und über einen Einlaufstern 27 zu einer Sterilisationsanordnung gebracht. In einem ersten Sektor I erfolgt zunächst über einen Ausfahrwinkel von 135° ein Herabsenken der Strahlfinger in das Innere der Behältnisse 10 bzw. ein Anheben der Behältnisse 10. In einem weiteren Sektor II werden die Strahlfinger wieder zurückgefahren bzw. die Behältnisse 10 werden abgesenkt. Während dieses Ausfahrens des Strahlfingers aus den Behältnissen 10 wird die Vorrichtung 1 aktiviert und auf diese Weise eine Innensterilisation der Behältnisse 10 vorgenommen.

Anschließend werden die Behältnisse über einen Auslaufstern 28 abtransportiert.

Fig. 7 zeigt eine schematische Darstellung des Umlaufrades 31. Dieses Umlaufrad 31 weist eine Vielzahl von erfindungsgemäßen Vorrichtungen 1 auf, die jeweils auf der gleichen Höhe angeordnet sind, man erkennt jedoch, dass die einzelnen Behältnisse 10 jeweils bis zu einer Maximalposition angehoben werden und auf diese Weise die Vorrichtung 1 bzw. der Strahlfinger in Abhängigkeit von der Drehstellung unterschiedlich tief in die Behältnisse 10 eingetaucht werden. Während dieses gesamten Vorgangs sind die erfindungsgemäßen Vorrichtungen 1 aktiviert und auf diese Weise wird die Innenwandung der Behältnisse 10 über die gesamte Höhe der Behältnisse 1 sterilisiert. Die Länge der Strahlfinger wird dabei so gewählt, dass auch eine effektive Sterilisation der Böden der Behältnisse 10 möglich ist.

Fig. 8 zeigt eine weitere Ausführungsform für eine erfindungsgemäße Vorrichtung. Bei dieser Vorrichtung ist der Bereich zwischen dem Innengehäuse 20 und dem Außengehäuse 16 derart gestaltet, dass sich zwei Gaskanäle 22a und 22b bilden. Der Gasstrom wird durch einen der beiden Kanäle 22a zugeführt und streift seitlich gleichmäßig über das Austrittsfenster 8. Durch den anderen Kanal 22b kann der Gasstrom wieder abgeführt werden.

Sämtliche in den Anmeldungsunterlagen offenbarten Merkmale werden als erfindungswesentlich beansprucht, sofern sie einzeln oder in Kombination gegenüber dem Stand der Technik neu sind.

## Patentansprüche

1. Vorrichtung (1) zum Sterilisieren von Behältnissen (10) mit einem Behandlungskopf (5), der ein Austrittsfenster (8) aufweist, durch welches Ladungsträger hindurchtreten können, mit einer Ladungsträgererzeugungsquelle, die Ladungsträger erzeugt und mit einer
Beschleunigungseinrichtung (6) welche die Ladungsträger in Richtung des Austrittsfensters (8) beschleunigt, **dadurch gekennzeichnet, dass** der Querschnitt des Behandlungskopfes (5) derart beschaffen ist, dass der Behandlungkopf (5) durch die Mündung des Behältnisses (10) führbar ist und dass die Beschleunigungseinrichtung (6) die Ladungsträger derart beschleunigt, dass die aus dem Austrittsfenster (8) austretenden Ladungsträger auf eine Innenwandung (15) des Behältnisses (10) richtbar sind.

2. Vorrichtung (1) nach Anspruch 1, **dadurch gekennzeichnet, dass** das Austrittsfenster (8) und die
Beschleunigungseinrichtung (6) innerhalb eines Außengehäuses (16) angeordnet sind und dieses Gehäuse (16) derart beschaffen ist, dass es durch die Mündung des Behältnisses (10) führbar ist.

3. Vorrichtung (1) nach wenigstens einem der vorangegangenen Ansprüche, **dadurch gekennzeichnet, dass** die Vorrichtung (1) von einer Abschirmungseinrichtung (18) umgeben ist.

4. Vorrichtung (1) nach wenigstens einem der vorangegangenen Ansprüche, **dadurch gekennzeichnet, dass** die Vorrichtung (1) ein Innengehäuse (20) aufweist, innerhalb dessen die Beschleunigungseinrichtung (6) angeordnet ist und dass das Außengehäuse (16) dieses Innengehäuse (20) umgibt.

5. Vorrichtung (1) nach Anspruch 4, **dadurch gekennzeichnet, dass** zwischen dem Außengehäuse (16) und dem Innengehäuse (20) ein sich bis zu dem Behandlungskopf (5) erstreckender Raum (22) gebildet wird, durch den ein Medium und insbesondere ein gasförmiges Medium führbar ist.

6. Vorrichtung (1) nach Anspruch 5, **dadurch gekennzeichnet, dass**, der Raum (22) einen oder mehrere Kanäle (22a, 22b) aufweist, durch die jeweils ein gasförmiges oder flüssiges Medium geleitet werden kann.

7. Vorrichtung (1) nach wenigstens einem der Ansprüche 5 bis 6, **dadurch gekennzeichnet, dass** das gasförmige Medium aus einer Gruppe von gasförmigen Medien ausgewählt ist, welche Helium, Stickstoff, Argon, Kohlendioxid, Mischungen hieraus oder dergleichen enthält.

8. Vorrichtung (1) nach Anspruch 6, **dadurch gekennzeichnet, dass** das flüssige Medium aus einer Gruppe von flüssigen Medien ausgewählt ist, welche Wasser, Öl, flüssigen Stickstoff oder dergleichen enthält.

9. Vorrichtung (1) nach wenigstens einem der vorangegangenen Ansprüche, **dadurch gekennzeichnet, dass** das Austrittsfenster (8) aus einem Material hergestellt ist, welches aus einer Gruppe von Materialien ausgewählt ist, welche Titan, Quarzglas, Diamant, Kombinationen hieraus und dergleichen enthält.

10. Vorrichtung (1) nach wenigstens einem der vorangegangene Ansprüche, **dadurch gekennzeichnet, dass** das Austrittsfenster (8) eine Dicke aufweist, welche zwischen 3 µm und 30. µm , bevorzugt zwischen 4 µm und 25 µm und besonders bevorzugt zwischen 5 µm und 20 µm liegt.

11. Vorrichtung nach wenigstens einem der vorangegangenen Ansprüche, **dadurch gekennzeichnet, dass** die Vorrichtung (1) eine Ablenkeinrichtung (7) zum Ablenken der Ladungsträger aufweist.

12. Vorrichtung (1) nach wenigstens einem der vorangegangenen Ansprüche, **dadurch gekennzeichnet, dass** der Behandlungskopf (5) gegenüber dem Behältnis (10) in einer Längsrichtung (L) des Behältnisses (10) bewegbar ist.

13. Vorrichtung (1) nach wenigstens einem der vorangegangenen Ansprüche, **dadurch gekennzeichnet, dass** der Behandlungskopf (5) gegenüber dem Behältnis (10) drehbar ist.

14. Vorrichtung (1) nach wenigstens einem der vorangegangenen Ansprüche, **dadurch gekennzeichnet, dass** der Behandlungskopf (5) in einer radialen Richtung des Behältnisses (10) bewegbar ist.

15. Anordnung (30) zum Behandeln von Behältnissen mit einer Vorrichtung (1) nach wenigstens einem der vorangegangenen Ansprüche.

16. Anordnung (30) nach Anspruch 15, **dadurch gekennzeichnet, dass** die Anordnung (30) eine Einrichtung (32) zum Befüllen von Behältnissen (10) aufweist und die Vorrichtung (1) stromaufwärts bezüglich dieser Einrichtung (32) angeordnet ist.

17. Anordnung (30) nach Anspruch 15, **dadurch gekennzeichnet, dass** die Anordnung eine Verschiebeeinrichtung (36) aufweist, welche die Behältnisse in der Längsrichtung der Behältnisse (10) gegenüber der Vorrichtung (1) verschiebt.

18. Anordnung (30) nach wenigstens einem der vorangegangenen Ansprüche 15 - 17, **dadurch gekennzeichnet, dass** die Anordnung (30) zwischen einer Expansionseinrichtung (38) für die Behältnisse und der Einrichtung (32) zum Befüllen der Behältnisse (10) angeordnet ist.

19. Anordnung (30) nach wenigstens einem der vorangegangenen Ansprüche 15 - 18, **dadurch gekennzeichnet, dass** die Anordnung (30) eine Vielzahl von Vorrichtungen (1) zum Sterilisieren von Behältnissen (10) nach wenigstens einem der vorangegangenen Ansprüche 1 - 14 aufweist, wobei diese Vorrichtungen (1) entlang einer Kreisbahn angeordnet sind.

20. Verfahren zum Sterilisieren von Behältnissen (10), wobei in einer Vorrichtung (1) zum Sterilisieren von Behältnissen (10) Ladungsträger erzeugt und in Richtung eines Austrittfensters (8), welches in einem Behandlungskopf (5) angeordnet ist, beschleunigt werden, **dadurch gekennzeichnet, dass** der Behandlungskopf (5) der Vorrichtung (1) durch eine Mündung des Behältnisses (10) in das Innere des Behältnisses (10) eingeführt wird und ausgehend von dem Behandlungskopf (5) beschleunigte Ladungsträger direkt auf die Innenwandung des Behältnisses (10) gerichtet werden und das Behältnis (10) relativ zu dem Behandlungskopf (5) bewegt wird.

21. Verfahren nach Anspruch 20, **dadurch gekennzeichnet, dass** die Ladungsträger vor Erreichen des Austrittsfensters (8) in einer radialen Richtung des Behältnisses (10) abgelenkt werden.

22. Verfahren nach Anspruch 20, **dadurch gekennzeichnet, dass** die Ladungsträger nach Austritt durch das Austrittsfenster (8) in einer radialen Richtung des Behältnisses (10) abgelenkt werden.

23. Verfahren nach Anspruch 20, **dadurch gekennzeichnet, dass** das Austrittsfenster (8) gekühlt wird.

24. Verfahren nach Anspruch 23, **dadurch gekennzeichnet, dass** als Kühlmedium ein gasförmiges und / oder ein flüssiges Medium verwendet wird.
